(19) Europäisches Patentamt
European Patent Office
Office européen des brevets

(11) EP 0 936 212 B1

(12) EUROPEAN PATENT SPECIFICATION

(45) Date of publication and mention of the grant of the patent:
**21.04.2004 Bulletin 2004/17**

(51) Int Cl.$^7$: **C07C 211/52**

(21) Application number: **99102538.8**

(22) Date of filing: **10.02.1999**

# (54) Fluorine-containing aniline compounds

Fluor enthaltende Anilin-Verbindungen

Composés d' aniline contenant du fluor

(84) Designated Contracting States:
**CH DE FR GB IT LI**

(30) Priority: **17.02.1998 JP 5135198**

(43) Date of publication of application:
**18.08.1999 Bulletin 1999/33**

(73) Proprietor: **NIHON NOHYAKU CO., LTD.**
**Chuo-ku Tokyo100-0027 (JP)**

(72) Inventors:
- **Tohnishi, Masanori**
  **Sakai-shi (JP)**
- **Kohno, Eiji**
  **Habikino-shi (JP)**
- **Nakao, Hayami**
  **Kawachinagano-shi (JP)**
- **Seo, Akira**
  **Hashimoto-shi (JP)**

(74) Representative: **Grünecker, Kinkeldey,**
**Stockmair & Schwanhäusser Anwaltssozietät**
**Maximilianstrasse 58**
**80538 München (DE)**

(56) References cited:
**EP-A- 0 246 061**
**EP-A- 0 298 803**
**GB-A- 1 535 234**
**US-A- 4 120 863**
**US-A- 5 248 781**

- **PATENT ABSTRACTS OF JAPAN vol. 018, no. 528 (C-1258), 6 October 1994 & JP 06 184065 A (SUMITOMO CHEM CO LTD), 5 July 1994**
- **CHEMICAL ABSTRACTS, vol. 109, no. 25, 19 December 1988 Columbus, Ohio, US; abstract no. 230409p, ZHOU QILIN ET AL.: "A facile method for fluoalkylation of aniline and its derivatives" page 803; column 1; XP002101167 & JOURNAL OF FLUORINE CHEMISTRY., vol. 39, no. 1, 1988, pages 87-98, LAUSANNE CH**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to a fluorine-containing aniline compound which is useful as an intermediate of medicinal, agrochemical or chemical products. In particular, it relates to a compound which is useful as a starting material for agricultural and horticultural insecticides as disclosed in Japanese Patent Application No. 9-339393.

JP-A-63-99046 and JP-A-6-184065 describe that anilines similar to the fluorine-containing aniline compound of the present invention are useful as intermediates of benzoylurea type insecticides.

GB-A-1535234 discloses fluorinated arylamines containing a pentafluoroethyl group which are useful as intermediates for the preparation of dye stuffs.

EP-A-0919542 discloses phthalic acid diamide derivatives and agricultural and horticultural insecticides containing said phthaldiamide derivatives.

It is the object of the present invention to provide fluorine-containing aniline compounds useful as intermediates of agricultural and horticultural insecticides.

Said object is achieved by a fluorine-containing aniline compound represented by the general formula (I -1):

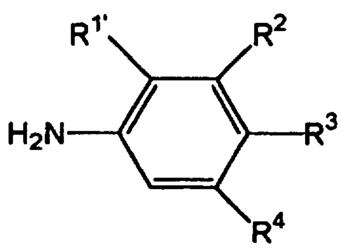

(I -1)

wherein $R^{1'}$ is a $C_1$-$C_6$ alkyl group, a $C_1$-$C_6$ alkoxy group or a trifluoromethyl group, and each of $R^2$, $R^3$ and $R^4$ is a hydrogen atom or a $C_2$-$C_6$ perfluoroalkyl group, provided that at least one of $R^2$, $R^3$ and $R^4$ is not a hydrogen atom; and that $R^3$ is not a pentafluoroethyl group, when $R^{1'}$ is a methyl group and each of $R^2$ and $R^4$ is a hydrogen atom.

It is preferred that $R^{1'}$ is a $C_1$-$C_6$ alkyl group.

The invention further relates to compounds represented by general formula (I-3)

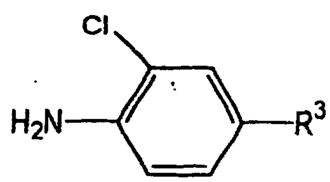

(I -3)

wherein $R^3$ is heptafluoropropane-2-yl or heptafluoropropane-1-yl.

Said object is further achieved by the use of a fluorine-containing aniline compound represented by the general formula (I -2):

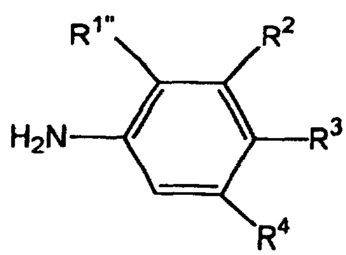

(I -2)

wherein $R^{1''}$ is a chlorine atom, a bromine atom, a $C_1$-$C_6$ alkyl group, a $C_1$-$C_6$ alkoxy group or a trifluoromethyl group, and each of $R^2$, $R^3$ and $R^4$ is a hydrogen atom or a $C_2$-$C_6$ perfluoroalkyl group, provided that at least one of $R^2$, $R^3$ and $R^4$ is not a hydrogen atom; and that $R^3$ is not a pentafluoroethyl group, when $R^{1''}$ is a methyl group and each of $R^2$ and $R^4$ is a hydrogen atom as intermediates of agricultural and horticultural insecticides.

It is preferred that $R^{1''}$ is a $C_1$-$C_6$ alkyl group.

The fluorine-containing aniline compound of the present invention can be produced, for example, by any of the following production processes.

Production process 1.

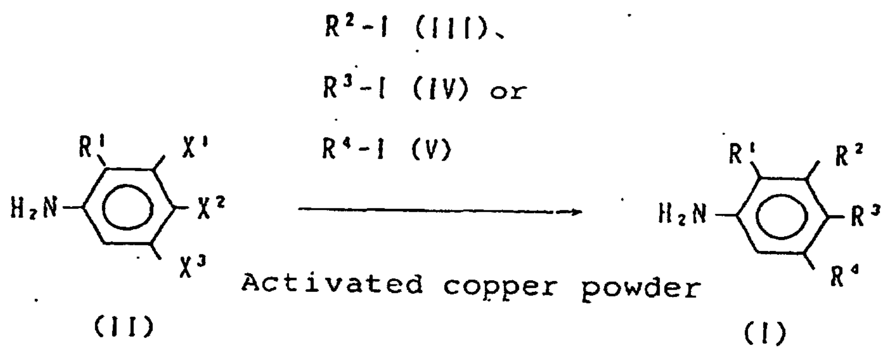


wherein each of $X^1$, $X^2$ and $X^3$ is a hydrogen atom, an iodine atom or a bromine atom, provided that at least one of $X^1$, $X^2$ and $X^3$ is not a hydrogen atom, $R^1$ is $R^{1'}$ or $R^{1''}$ which are as defined above, and $R^2$, $R^3$ and $R^4$ are as defined above.

A fluorine-containing aniline compound of the general formula (I) can be produced by reacting an aniline derivative of the general formula (II) with a perfluoroalkyl iodide of the general formula (III), general formula (IV) or general formula (V) in the presence of activated copper powder and an inert solvent.

Usually, the amount of each of the perfluoroalkyl iodide of the general formula (III), (IV) or (V) and the activated copper powder used may be properly chosen in the range of 1 to 5 times the amount of the aniline derivative of the general formula (II).

As the inert solvent used in the reaction, any solvent may be used as long as it does not remarkably inhibit the progress of the reaction. There can be used, for example, aprotic polar solvents such as N,N-dimethylformamide (DMF), dimethyl sulfoxide (DMSO), etc. The reaction temperature may be properly chosen in the range of room temperature to 200°C.

After completion of the reaction, the desired compound is isolated from the reaction mixture containing the desired compound by a conventional method and can, if necessary, be purified by silica gel chromatography, distillation, recrystallization, etc.

The reaction can be carried out by the method described in Bull. Chem. Soc. Jpn., 65, 2141-2144 (1992).

Production process 2.

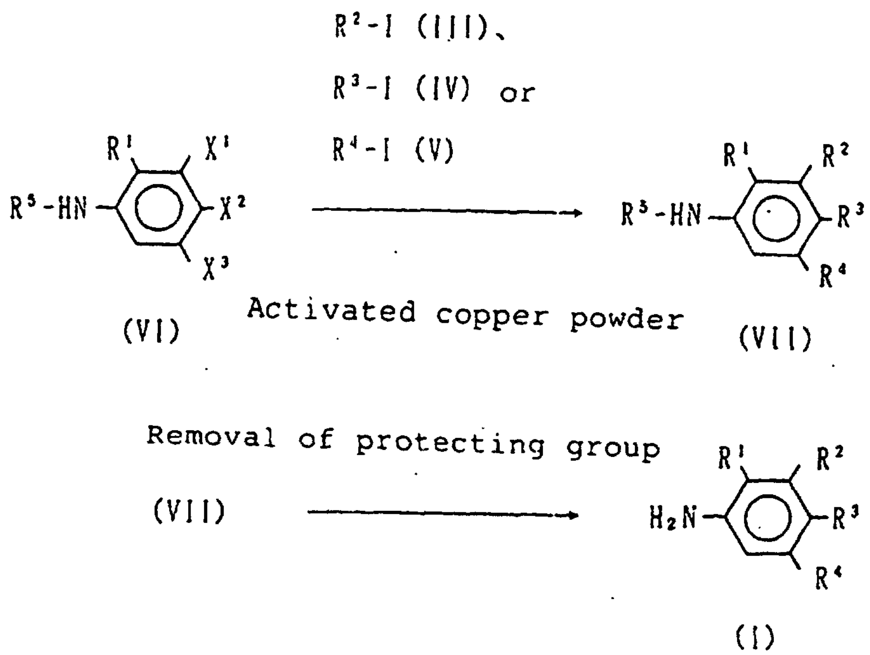


wherein $R^1$, $R^2$, $R^3$, $R^4$, $X^1$, $X^2$ and $X^3$ are as defined above, and $R^5$ is a protecting group such as an acyl group.

A fluorine-containing aniline compound of the general formula (I) can be produced by reacting an acylaniline derivative of the general formula (VI) with a perfluoroalkyl iodide of the general formula (III), general formula (IV) or general formula (V) in the presence of activated copper powder and an inert solvent to obtain an acylaniline derivative of the general formula (VII), and deacylating the acylaniline derivative (VII) after or without isolation.

① General formula (VI) → general formula (VII)

This reaction can be carried out according to production process 1.

② General formula (VII) → general formula (I)

This reaction is usually carried out under acidic conditions. In the reaction, for example, an aqueous mineral acid solution such as a 5 to 35% aqueous hydrochloric acid solution is used as an acid. If necessary, there may be used together therewith alcohols such as methanol, ethanol, etc., and inert solvents such as tetrahydrofuran (THF), acetonitrile, etc. The reaction temperature may be chosen in the range of room temperature to the boiling point of the solvent used. The desired compound can be produced by carrying out the similar workup as in production process 1.

Production process 3.

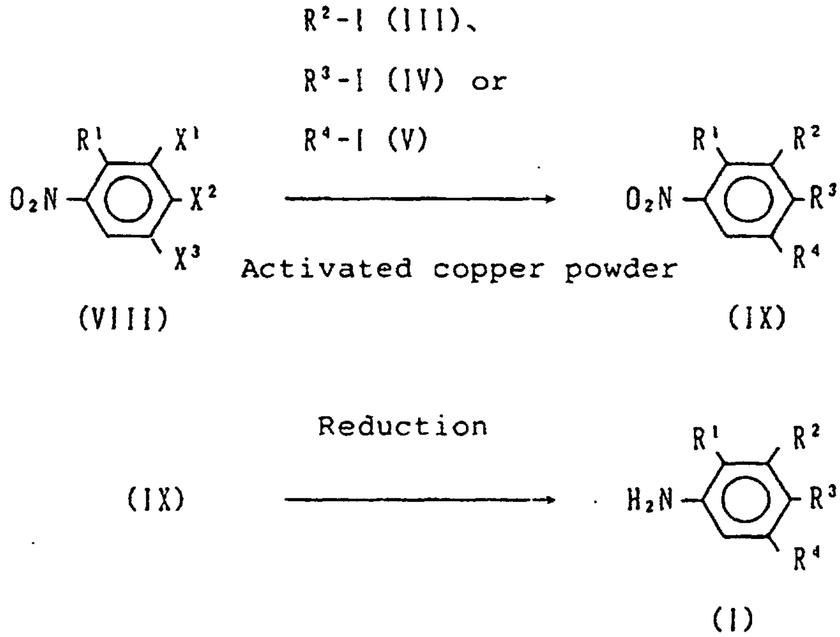


wherein $R^1$, $R^2$, $R^3$, $R^4$, $X^1$, $X^2$ and $X^3$ are as defined above.

A fluorine-containing aniline compound of the general formula (I) can be produced by reacting a nitrobenzene derivative of the general formula (VIII) with a perfluoroalkyl iodide of the general formula (III), general formula (IV) or general formula (V) in the presence of activated copper powder and an inert solvent to obtain a nitrobenzene derivative of the general formula (IX), and reducing the nitrobenzene derivative (IX) after or without isolation.

① General formula (VIII) → general formula (IX)

This reaction can be carried out according to production process 1.

② General formula (IX) → general formula (I)

Usually, the desired compound can be produced by subjecting the nitrobenzene derivative (IX) to catalytic hydrogenation in an alcohol solvent in the presence of a catalyst such as 5% palladium carbon (proportion: 1 to 5% by weight), or reducing the nitrobenzene derivative (IX) with an aqueous hydrochloric acid solution containing stannous chloride, in a solvent such as ethanol.

The reaction ② can be carried out according to the method described in Journal of Chemical Society of Japan, 1973, 2351.

Typical examples of the fluorine-containing aniline compound of the general formula (I) of the present invention are given in Table 1 but they are not intended in any way to limit the scope of the present invention.

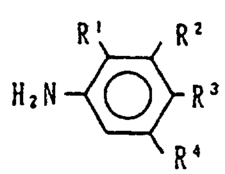

(I)

Table 1

| No. | $R^1$ | $R^2$ | $R^3$ | $R^4$ | Physical property or $^1H$-NMR(δ, ppm) |
|---|---|---|---|---|---|
| 1 | $CH_3$ | H | H | $C_2F_5$ | 2.21(s, 3H), 3.84(br, 2H), 6.86(s, 1H), 6.91(d, 1H), 7.15(d, 1H). |
| 2 | $CH_3$ | N | H | $i\text{-}C_3F_7$ | |
| 3 | $CH_3$ | H | $i\text{-}C_3F_7$ | H | b.p. 100-110°C/10mmHg |
| 4 | $CH_3$ | H | $n\text{-}C_3F_7$ | H | 2.20(s, 3H), 3.9(br, 2H), 6.70(d, 1H), 7.22-7.27(m, 2H). |
| 5 | $CH_3$ | N | $n\text{-}C_4F_9$ | H | 2.19(s, 3H), 4.2(br, 2H), 6.70(d, 1H), 7.20-7.26(m, 2H). |
| 6 | $CH_3$ | H | $CF(CF_3)\text{-}C_2F_5$ | H | |
| 7 | $CH_3$ | $C_2F_5$ | H | H | 2.22(s. 3H), 3.83(br, 2H), 6.86(d, 1H), 6.99(d, 1H), 7.12(t, 1H). |
| 8 | $CH_3$ | $i\text{-}C_3F_7$ | H | H | |
| 10 | Cl | H | $C_2F_5$ | H | 4.4(br, 2H), 6.8(d, 1H), 7.27(dd, 1H), 7.47(d, 1H). |
| 11 | Cl | H | $i\text{-}C_3F_7$ | H | 4.5(br, 2H), 7.41(s, 1H), 7.81(d, 1H), 8.05(d, 1H). |
| 12 | Cl | H | $n\text{-}C_3F_7$ | H | 4.1(br, 2H), 6.83(d, 1H), 7.24(d, 1H), 7.43(s, 1H). |
| 13 | Br | H | $C_2F_5$ | H | |
| 14 | $OCH_3$ | H | $C_2F_5$ | H | 3.85(br, 2H), 3.93(s, 3H), 6.72(d, 1H), 6.92(s, 1H, 7.03(d, 1H). |
| 15 | $OCH_3$ | H | $i\text{-}C_3F_7$ | H | |
| 16 | $OC_2H_5$ | H | $i\text{-}C_3F_7$ | H | |
| 17 | $C_2H_5$ | H | $C_2F_5$ | H | 1.29(t, 3H), 2.52(q, 2H), 3.95(br, 2H), 6.8(d, 1H), 7.2-7.26(m, 2H). |
| 18 | $C_2H_5$ | H | $i\text{-}C_3F_7$ | H | |
| 19 | $i\text{-}C_3F_7$ | H | $i\text{-}C_3F_7$ | H | |
| 20 | $n\text{-}C_4H_9$ | H | $C_2F_5$ | H | 0.9(t, 3H), 1.4(q, 2H), 1.6(q, 2H), 2.5(t, 2H), 3.95(br, 2H), 6.69(d, 2H), 7.19-7.21(m, 2H). |
| 21 | $CF_3$ | H | $C_2F_5$ | H | 4.52(br, 2H), 6.81(d, 1H), 7.46(d, 1H), 7.63(br, 1H). |
| 22 | $CF_3$ | H | $i\text{-}C_3F_7$ | H | |

EXAMPLES

Typical examples and reference examples of the present invention are described below, but they should not be construed as limiting the scope of the invention.

Example 1

1-1. Production of 2-ethyl-4-pentafluoroethylacetanilide

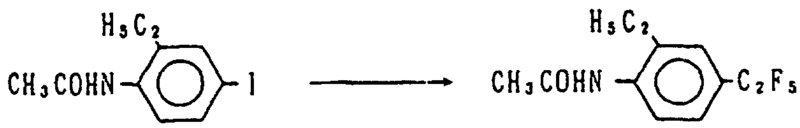

In an autoclave were placed 4.0 g (0.0138 mole) of 2-ethyl-4-iodoacetanilide, 1.8 g of copper powder, 5.1 g of iodopentafluoroethane and 40 ml of DMSO, and the reaction was carried out with stirring for 6 hours while maintaining the internal temperature at 120°C. After completion of the reaction, the reaction mixture was cooled to room temperature, poured into 200 ml of ice water, and thoroughly stirred. Then, the insoluble materials were removed and the desired compound was extracted with 200 ml of ethyl acetate. The extract solution was washed with water, dried over anhydrous sodium sulfate and then concentrated, after which the residue was purified by a silica gel column chromatography to obtain 0.7 g (yield: 18%) of the desired compound.

1-2. Production of 2-ethyl-4-pentafluoroethylaniline (compound No. 17)

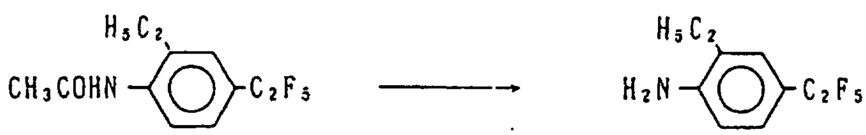

To 10 ml of a 6N aqueous hydrochloric acid solution was added 0.6 g (2.1 mmoles) of 2-ethyl-4-pentafluoroethylacetanilide, and the reaction was carried out with heating under reflux for 2 hours. After completion of the reaction, the reaction solution was ice-cooled and then neutralized with a 10% aqueous sodium hydroxide solution, and the desired compound was extracted with ethyl acetate. The extract solution was washed with water, dried over anhydrous sodium sulfate and then concentrated under reduced pressure to obtain 0.4 g of the desired compound.

Physical property:

| | |
|---|---|
| $^1$H-NMR (δ, ppm) | 1.29(t, 3H), 2.52(q, 2H), 3.95(br, 2H), 6.8(d, 1H), 7.2-7.26(m, 2H). |
| Yield: | 80%. |

Example 2

Production of 2-chloro-4-pentafluoroethylaniline (compound No. 10)

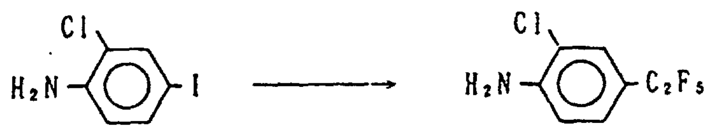

In an autoclave were placed 5.0 g (19.7 mmoles) of 2-chloro-4-iodoaniline, 2.8 g of copper powder, 10.0 g of iodopentafluoroethane and 50 ml of DMF, and the reaction was carried out with stirring for 20 hours while maintaining the internal temperature at 135°C. After completion of the reaction, the reaction mixture was cooled to room temperature, poured into 200 ml of ice water, and thoroughly stirred. Then, the insoluble materials were removed and the desired compound was extracted with 200 ml of ethyl acetate. The extract solution was washed with water, dried over anhydrous sodium sulfate and then concentrated, after which the residue was purified by a silica gel column chromatography to obtain 4.2 g of the desired compound.

Physical property:

| | |
|---|---|
| $^1$H-NMR (δ, ppm) | 4.4(br, 2H), 6.8(d, 1H), 7.27(dd, 1H), 7.47(d, 1H). |
| Yield: | 87%. |

Example 3

Production of 2-trifluoromethyl-4-pentafluoroethylaniline (compound No. 21)

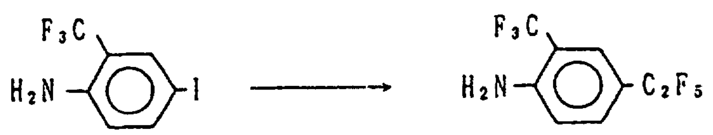

In an autoclave were placed 6.0 g (20.9 moles) of 2-trifluoro-4-iodoaniline, 2.8 g of copper powder, 11.1 g of iodopentafluoroethane and 40 ml of DMF, and the reaction was carried out with stirring for 7 hours while maintaining the internal temperature at 135°C. After completion of the reaction, the reaction mixture was cooled to room temperature, poured into 200 ml of ice water, and thoroughly stirred. Then, the insoluble materials were removed and the desired compound was extracted with 200 ml of ethyl acetate. The extract solution was washed with water, dried over anhydrous sodium sulfate and then concentrated, after which the residue was purified by a silica gel column chromatography to obtain 3.9 g of the desired compound.

Physical property:

| | |
|---|---|
| $^1$H-NMR (δ, ppm) | 4.52(br, 2H), 6.81(d, 1H), 7.27(dd, 1H), 7.48(d, 1H), 7.63(br, 1H). |
| Yield: | 67%. |

Example 4

4-1. Production of 2-methyl-4-(heptafluoropropan-2-yl)-nitrobenzene

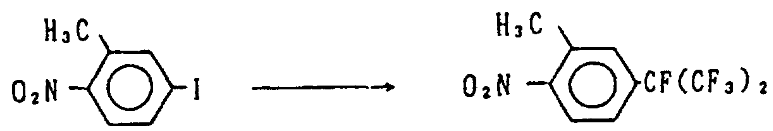

In an autoclave were placed 12.0 g (0.0456 mole) of 4-iodo-2-methylnitrobenzene, 11.6 g of copper powder, 40 g of 2-iodoheptafluoropropane and 200 ml of DMF, and the reaction was carried out with stirring for 6 hours while maintaining the internal temperature at 140°C. After completion of the reaction, the reaction mixture was cooled to room temperature, poured into 600 ml of ice water, and thoroughly stirred. Then, the insoluble materials were removed and the desired compound was extracted with 300 ml of hexane. The extract solution was washed with water, dried over anhydrous sodium sulfate and then concentrated, after which the residue was purified by vacuum distillation to obtain 11.4 g of the desired compound.

Physical property: b.p. 120 - 125°C/10 mmHg.

Yield: 82%.

4-2. Production of 2-methyl-4-(heptafluoropropan-2-yl)-aniline (compound No. 3)

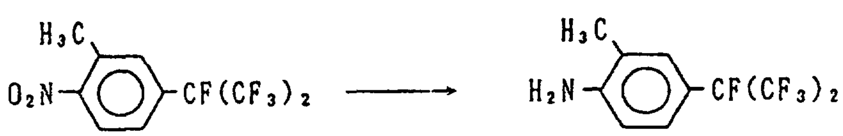

In 60 ml of ethanol was dissolved 11.4 g (0.0374 mole) of 2-methyl-4-(heptafluoropropan-2-yl)-nitrobenzene, and a solution of 29.5 g of $SnCl_2 \cdot 2H_2O$ in 40 ml of hydrochloric acid was added dropwise thereto under ice-cooling over a period of 30 minutes. After completion of the dropwise addition, the reaction was carried out at room temperature for 2 hours. After completion of the reaction, the reaction solution was poured into 200 ml of ice water and neutralized with a 40% aqueous sodium hydroxide solution under ice-cooling. Then, a 40% aqueous sodium hydroxide solution was further added thereto until a homogeneous solution was obtained, and the desired compound was extracted with 100 ml of ether. The extract solution was washed with water, dried over anhydrous sodium sulfate and then concentrated, after which the residue was purified by vacuum distillation to obtain 9.8 g of the desired compound.

Physical property: b.p. 100 - 110°C/10 mmHg.

Yield: 95%.

Reference Example 1

Production of 3-bromo-$N^1$-(4-nonafluorobutyl-2-methylphenyl)-$N^2$-isopropyl-phthalic acid diamide (hereinafter referred to as "reference compound")

In 10 ml of tetrahydrofuran was dissolved 0.54g of 6-bromo-N-isopropyl-phthalic acid isoimide, followed by adding thereto 0.30 g of 2-methyl-4-nonafluorobutylaniline, and the reaction was carried out with stirring for 1 hour. After completion of the reaction, the solvent was removed from the reaction solution by distillation under reduced pressure, and the resulting residue was washed with ether-n-hexane to obtain 1.1 g of the desired compound.

Physical property: m.p. 190 - 191°C. Yield 94%.

Reference Example 2

Insecticidal effect on diamondback moth (Plutella xylostella)

Adult diamondback moths were released and allowed to oviposit on a Chinese cabbage seedling. Two days after the release, the seedling having eggs deposited thereon was immersed for about 30 seconds in a liquid chemical prepared by diluting a preparation containing the reference compound as an active ingredient to adjust the concentration to 500 ppm. After air-drying, the seedling was allowed to stand in a room thermostated at 25°C. Six days after the immersion, the hatched insects were counted and the mortality was calculated according to the following equation. The test was carried out with three replications.

$$\text{Corrected mortality (\%)} = \frac{\left[\text{Number of hatched insects in untreated group}\right] - \left[\text{Number of hatched insects in treated group}\right]}{\left[\text{Number of hatched insects in untreated group}\right]} \times 100$$

As a result, the reference compound was found to have a mortality of 100%.

Reference Example 3

Insecticidal effect on common cutworm (Spodoptera litura)

A piece of cabbage leaf (cultivar: Shikidori) was immersed for about 30 seconds in a liquid chemical prepared by diluting a preparation containing the reference compound as an active ingredient to adjust the concentration to 500 ppm. After air-drying, the piece was placed in a plastic Petri dish with a diameter of 9 cm whose bottom had been covered with a wetted filter paper. The piece was inoculated with third-instar larvae of common cutworm and the Petri dish was allowed to stand in a room thermostated at 25°C and having a relative humidity of 70%. Four days after the inoculation, the dead and alive were counted and the mortality was calculated according to the equation described in Reference Example 2. The test was carried out with three replications of 10 insects.

As a result, the reference compound was found to have a mortality of 100%.

**Claims**

1. A fluorine-containing aniline compound represented by the general formula (I -1):

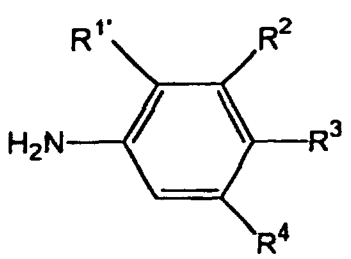

(I -1)

wherein $R^{1'}$ is a $C_1$-$C_6$ alkyl group, a $C_1$-$C_6$ alkoxy group or a trifluoromethyl group, and each of $R^2$, $R^3$ and $R^4$ is a hydrogen atom or a $C_2$-$C_6$ perfluoroalkyl group, provided that at least one of $R^2$, $R^3$ and $R^4$ is not a hydrogen

atom; and that $R^3$ is not a pentafluoroethyl group, when $R^{1'}$ is a methyl group and each of $R^2$ and $R^4$ is a hydrogen atom.

**2.** The fluorine-containing aniline compound according to claim 1, wherein $R^{1'}$ is a $C_1$-$C_6$ alkyl group.

**3.** A fluorine-containing aniline compound represented by general formula (I -3):

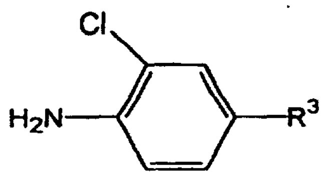

(I -3)

wherein $R^3$ is heptafluoropropane-2-yl or heptafluoropropane-1-yl.

**4.** Use of a fluorine-containing aniline compound represented by the general formula (I -2):

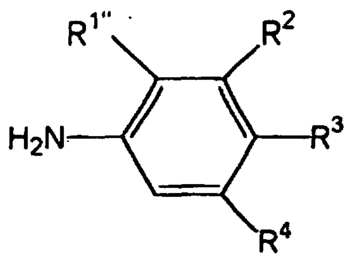

(I –2)

wherein $R^{1''}$ is a chlorine atom, a bromine atom, a $C_1$-$C_6$ alkyl group, a $C_1$-$C_6$ alkoxy group or a trifluoromethyl group, and each of $R^2$, $R^3$ and $R^4$ is a hydrogen atom or a $C_2$-$C_6$ perfluoroalkyl group, provided that at least one of $R^2$, $R^3$ and $R^4$ is not a hydrogen atom; and that $R^3$ is not a pentafluoroethyl group, when $R^{1''}$ is a methyl group and each of $R^2$ and $R^4$ is a hydrogen atom as intermediates of agricultural and horticultural insecticides.

**5.** The use of a fluorine-containing aniline compound according to claim 4, wherein $R^{1''}$ is a $C_1$-$C_6$ alkyl group.

**Patentansprüche**

**1.** Fluor-enthaltende Anilinverbindung gemäß der allgemeinen Formel (I -1):

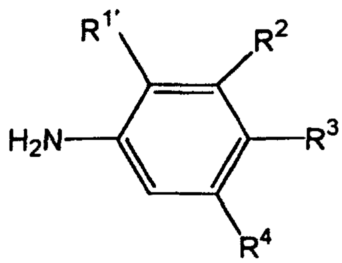

(I-1)

worin $R^{1'}$ eine $C_1$-$C_6$-Alkylgruppe, eine $C_1$-$C_6$-Alkoxygruppe oder eine Trifluormethylgruppe ist, und jedes von $R^2$, $R^3$ und $R^4$ ein Wasserstoffatom oder eine $C_2$-$C_6$-Perfluoralkylgruppe ist, vorausgesetzt, dass zumindest eines von $R^2$, $R^3$ und $R^4$ kein Wasserstoffatom ist; und dass $R^3$ keine Pentafluorethylgruppe ist, wenn $R^{1'}$ eine Methylgruppe und jedes von $R^2$ und $R^4$ ein Wasserstoffatom ist.

**2.** Fluor-enthaltende Anilinverbindung gemäß Anspruch 1, worin $R^{1'}$ eine $C_1$-$C_6$-Alkylgruppe ist.

3. Fluor-enthaltende Anilinverbindung gemäß der allgemeinen Formel (I -3):

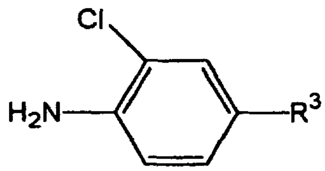

(I -3)

worin $R^3$ Heptafluorpropan-2-yl oder Heptafluorpropan-1-yl ist.

4. Verwendung der Fluor-enthaltenden Anilinverbindung gemäß der allgemeinen Formel (I -2):

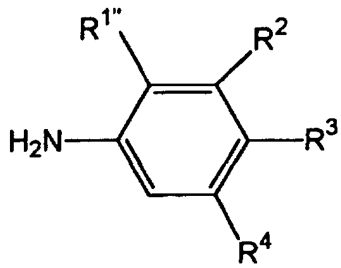

(I-2)

worin $R^{1''}$ ein Chloratom, ein Bromatom, eine $C_1$-$C_6$-Alkylgruppe, eine $C_1$-$C_6$-Alkoxygruppe oder eine Trifluormethylgruppe ist, und jedes von $R^2$, $R^3$ und $R^4$ ein Wasserstoffatom oder eine $C_2$-$C_6$-Perfluoralkylgruppe ist, vorausgesetzt, dass zumindest eines von $R^2$, $R^3$ und $R^4$ kein Wasserstoffatom ist; und dass $R^3$ nicht eine Pentafluorethylgruppe ist, wenn $R^{1''}$ eine Methylgruppe und jedes von $R^2$ und $R^4$ ein Wasserstoffatom ist,
als Zwischenprodukte von Landwirtschafts- und Gartenbauinsektiziden.

5. Verwendung der Fluor-enthaltenden Anilinverbindung gemäß Anspruch 4, worin $R^{1''}$ eine $C_1$-$C_6$-Alkylgruppe ist.

**Revendications**

1. Composé d'aniline contenant du fluor représenté par la formule générale (I-1) :

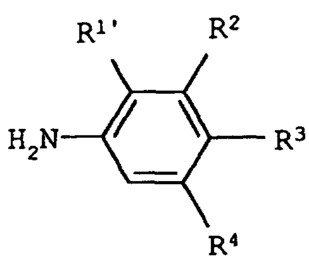

(I-1)

dans laquelle $R^{1'}$ est un groupe alkyle en $C_1$ à $C_6$, un groupe alcoxy en $C_1$ à $C_6$ ou un groupe trifluorométhyle, et chacun des $R^2$, $R^3$ et $R^4$ est un atome hydrogène ou un groupe perfluoroalkyle en $C_2$ à $C_6$, à condition qu'au moins un des $R^2$, $R^3$ et $R^4$ ne soit pas un atome d'hydrogène; et que $R^3$ ne soit pas un groupe pentafluoroéthyle, quand $R^{1'}$ est un groupe méthyle et que chacun des $R^2$ et $R^4$ est un atome d'hydrogène.

2. Composé d'aniline contenant du fluor selon la revendication 1, dans lequel $R^{1'}$ est un groupe alkyle en $C_1$ à $C_6$.

**3.** Composé d'aniline contenant du fluor représenté par la formule générale (I-3) :

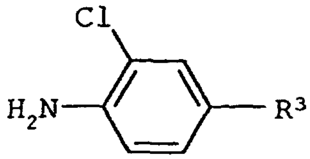

(I-3)

dans laquelle $R^3$ est un heptafluoropropan-2-yle ou un heptafluoropropan-1-yle.

**4.** Utilisation d'un composé d'aniline contenant du fluor représenté par la formule générale (I-2) :

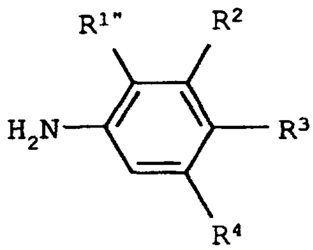

(I-2)

dans laquelle $R^{1"}$ est un atome de chlore, un atome de brome, un groupe alkyle en $C_1$ à $C_6$, un groupe alcoxy en $C_1$ à $C_6$ ou un groupe trifluorométhyle, et chacun des $R^2$, $R^3$ et $R^4$ est un atome d'hydrogène ou un groupe perfluoroalkyle en $C_2$ à $C_6$, à condition qu'au moins un des $R^2$, $R^3$ et $R^4$ ne soit pas un atome d'hydrogène; et que $R^3$ ne soit pas un groupe pentafluoroéthyle, quand $R^{1"}$ est un groupe méthyle et que chacun des $R^2$ et $R^4$ est un atome d'hydrogène en tant qu'intermédiaires d'insecticides agricoles et horticoles.

**5.** Utilisation d'un composé d'aniline contenant du fluor selon la revendication 4, dans lequel $R^{1"}$ est un groupe alkyle en $C_1$ à $C_6$.